# EUROPEAN PATENT SPECIFICATION

(11) **EP 1 940 548 B1**
(45) Date of publication and mention of the grant of the patent: **10.10.2012**
(21) Application number: 06795015.4
(22) Date of filing: 27.10.2006
(51) Int. Cl.: B01L 1/02, B25J 21/02, C12M 1/00, C12M 1/18

(54) **LABORATORY APPARATUS WITH INCUBATOR**
LABORVORRICHTUNG MIT INKUBATOR
APPAREIL DE LABORATOIRE

(30) Priority: 27.10.2005 GB 0521884
(43) Date of publication of application: 09.07.2008
(73) Proprietor: Newcastle-Upon-Tyne Hospitals NHS Trust, Newcastle-upon-Tyne Tyne and Wear NE7 7DN (GB)
(72) Inventor: HERBERT, Mary, Tyne & Wear NE1 4EP (GB); HARBOTTLE, Stephen, Tyne & Wear NE1 4EP (GB); FENWICK, Jeanette, Tyne & Wear NE1 4EP (GB); WALKER, James, Derbyshire SK13 8PT (GB)
(74) Representative: Archer, Graham John
(86) International application number: PCT/GB2006/050358
(87) International publication number: WO 2007/049078

(56) References cited:
- WO-A-01/30962
- WO-A-96/11092
- FR-A1- 2 605 016
- US-A1- 4 696 902

## Description

### Field of the Invention

The invention relates to laboratory apparatus; in particular to laboratory apparatus for use in an In Vitro Fertilisation treatment or stem cell derivation laboratory.

### Background of the Invention

In vitro manipulation of sensitive biological materials, such as oocytes, sperm or embryos, requires highly controlled environments. To increase the chances of success in procedures such as embryonic stem cell derivation or in vitro fertilisation, the air quality in which the various processes are carried out must be at an optimum and disturbances to cultures must be kept to a minimum.

In addition to controlling the actual environment, strict controls are also necessary in terms of checking, tracking, and minimising errors during the procedures.

In Vitro Fertilisation (or IVF) is a technique available to infertile couples to improve their chances of reproduction. The female is treated with hormones in order that a large number of unfertilised eggs or oocytes are produced. These oocytes are then extracted from the patient and subsequently fertilised in a laboratory, either by mixing with sperm or, in cases of severe male infertility, by injection of sperm into individual oocytes using the ICSI procedure (Intracytoplasmic sperm injection).

Fertilised oocytes, are then transferred to an incubator and kept in a monitored environment for 2-5 days to allow the embryo to develop. Traditionally one incubator will contain cultures from many different couples. A maximum of three, normally two of the best embryos are then selected and implanted into the female.

Females receiving IVF treatment sometimes agree to donate embryos for stem cell research. Stem cells can be derived from these embryos in a laboratory. The donated blastocysts are incubated in a culture medium, which is changed at twenty-four hour intervals for up to twelve days until putative embryonic stem cell colonies form. Colonies are transferred to further culture dishes and once there are sufficient colonies some may be frozen and stored.

New legislation will require IVF procedures to be carried out in environment with an air quality of grade A or as close to Grade A as possible. Currently these procedures are typically carried out in open fronted cabinets in standard laboratories. One way of meeting the grade A air requirement would be for all IVF procedures to be carried out in a specialist clean room facility. Staff working in a clean room must wear protective clothing to ensure that they do not contaminate the environment inside the room. Clean room clothing requirements include the use of protective suits, hoods, masks, gloves, and boots. Implementing and maintaining a clean room facility for IVF laboratories would make the technique much more time consuming and much more expensive to carry out.

During the IVF procedure, it is also vital that the correct sperm is matched with the correct oocytes, and that the correct embryo is then transplanted into the correct female. IVF laboratories will typically treat several couples per day and there are several opportunities for error in this process. Any mistakes are likely to have a huge impact on the future parents.

Software systems do exist to try and combat this problem. A computer program known as IVF Witness™ aims to eliminate human error in IVF treatment and to guarantee the correct parentage for babies bom through IVF. The system uses Radio Frequency Identification (RFID) technology to track labelled sample containers within a work area. Samples must be placed on a reader to be read and if samples within a work area are not from the same family, the computer system will flag a visual or audible warning to laboratory personnel. This system is a passive checking system as it does not actually prevent two sample containers from different families coming into contact, and there still exists margin for error.

WO 2004/003131 describes a system for ensuring matching between biological components such as oocytes and sperm. Sample containers containing the components are labelled with machine readable identity marks, and matching components have matching sets of identification marks. Each label is scanned prior to commencing a procedure to ensure that the components are matching. As with the IVF Witness™ system, this system is a passive checking system and it does not actually prevent two mis-matching components coming into contact.

Laboratory apparatus for use in IVF treatment is described in WO 2005/040330. This apparatus seeks to address the problem of disturbing cultures within an incubator each time an incubator is opened to add or remove other cultures. The apparatus comprises two cabinets connected by a passageway. Oocyte preparation and fertilisation is carried out in the first cabinet, which has an open front wall. Filtered air is circulated within the first cabinet. The fertilised oocytes are then transferred to the second cabinet which is substantially sealed and has a controlled atmosphere. Any cultures which are added or removed from the second cabinet have to pass through the passageway via the first cabinet so the environment in side the second cabinet is not directly exposed to the room environment. This set up of apparatus does not address the problem of sample cross-contamination, or sample mis-identification.

These known systems do not provide a suitable system which addresses the requirements for a clean air environment and the security requirements for a laboratory dealing with human tissue such as an IVF laboratory.

The present invention offers a system that mitigates the above-identified problems.

### Summary of the Invention

One aspect of the invention provides laboratory apparatus as specified in Claim 1.

Another aspect of the invention provides a method for controlling the processing of biological components as specified in Claim 23.

Preferred aspects of the invention are specified in the claims dependent on Claim 1 and Claim 23.

The invention provides a network or chain of isolation cabinets which are connected by incubator units which act as transfer hatches between cabinets. This network of cabinets provides a closed, controlled, clean air environment for the handling and manipulation of human tissue during IVF treatment or stem cell derivation. This chain of isolation cabinets enables these procedures to be carried out in a clean air environment without the need for a specialist clean room facility.

Incubator units are also provided with discrete compartments so that samples from one family are kept isolated from those of other families in the same laboratory.

The system also tracks sperm, eggs and embryos at each stage of the process creating a blind traceability system. The matching of sperm, oocytes, stem cells, embryos and patients or donors is controlled, significantly reducing the risk of misidentification of patient samples.

### Brief Description of the Drawings

In the drawings, which illustrate preferred embodiments of the invention:
Figure 1 is a plan view of a network of isolation cabinets;
Figure 2 is a schematic illustration of an isolation cabinet connected to an incubator, transfer hatch and control unit;
Figure 3 is a detailed illustration of the incubator shown in Figure 2;
Figure 4 is a flow diagram which illustrates the main operational steps when bringing a sample into an isolation cabinet through a transfer hatch; and
Figure 5 is a flow diagram which illustrates the main operational steps when bringing a sample into an isolation cabinet from an incubator.

### Detailed Description of the Preferred Embodiments

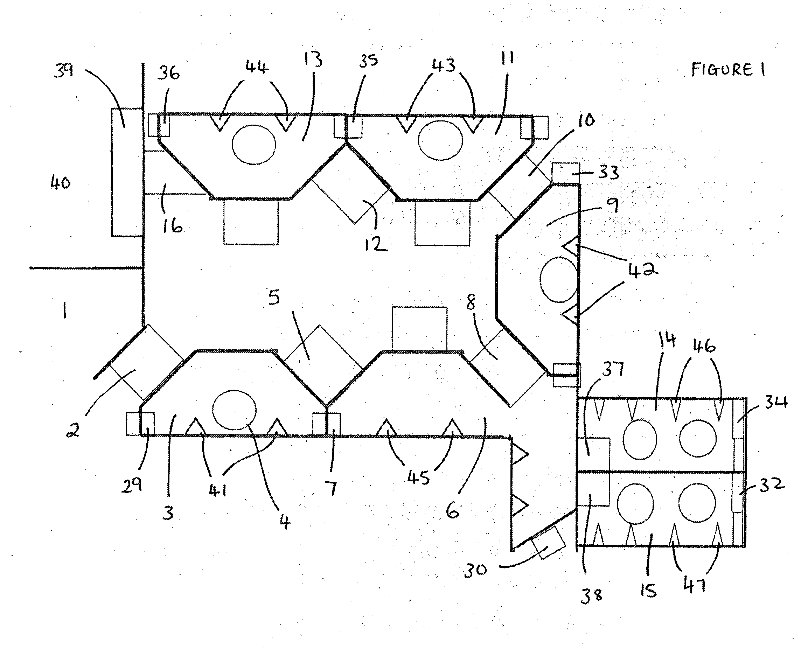

With reference to Figure 1, there is shown a plan view of laboratory apparatus for use in IVF or stem cell derivation comprising a horse shoe shaped chain of isolation cabinets 3, 6, 9, 11, 13, 14 and 15 connected by incubator units 5, 8, 10, 12, 16, 37 and 38.

Isolation cabinets 3, 6, 9, 11, 13, 14 and 15 are each substantially sealed cabinets, containing a viewing window and openings 41, 42, 43, 44, 44, 45, 46 and 47 to which gloves are attached to allow objects inside the cabinet to be handled. Each cabinet is supplied with air which has been filtered through a HEPA (High Efficiency Particulate Air) filter to reduce risk of contamination by airborne particles and activated carbon filters to prevent a build up of volatile compounds. The composition of the air is controlled so as to maintain the optimum amount of carbon dioxide, oxygen and nitrogen required. For example, in the IVF process the optimum amounts are carbon dioxide (approximately 5%), oxygen (approximately 5%) and nitrogen (approximately 90%). Each cabinet may contain equipment such as a microscope 4. Microscope eyepieces protrude through the front viewing window so that the equipment can be used whilst maintaining the cabinet seal.

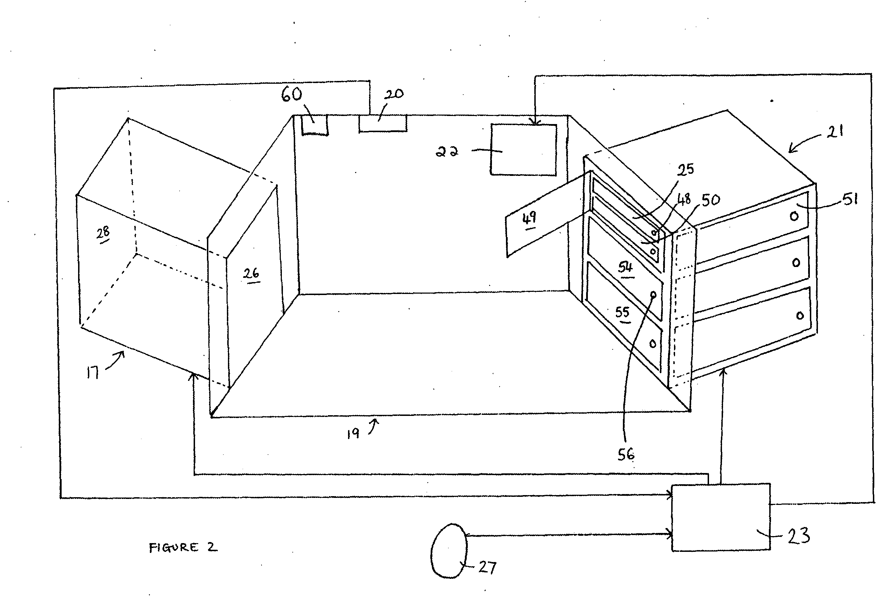

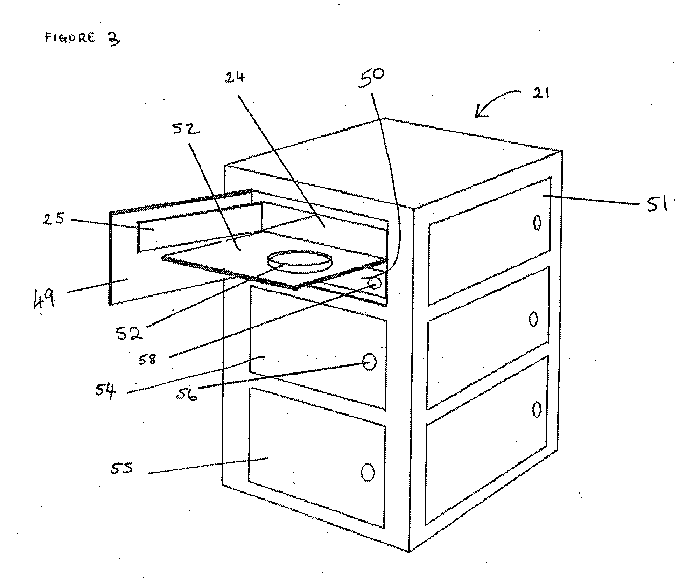

Incubator units 5, 8, 10, 12, 37 and 38 each connect two isolation cabinets. As shown in Figures 2 and 3, each incubator unit comprises six discrete compartments 24, each with independent access doors or openings 25. Each compartment of each incubator is accessible from either of the two connected cabinets. For example, compartment 24 is accessible either through outer door 49 in the left hand cabinet, or through outer door 51 in the right hand cabinet. The left hand side of incubator 21 comprises three outer doors 49, 54 and 55 each of which leads to two incubator compartments. Each incubator compartment is accessible by an independent inner door. For example outer door 49 leads to two inner doors 25 and 50. Inside each compartment there is a tray 52 mounted on a sliding mechanism. An operator slides tray 52 out of compartment 24 in order to either remove samples 52 from compartment 57 or to place samples 52 into compartment 24. Each compartment is assigned to a particular donor / patient family or couple undergoing treatment. Each compartment is independent and there is no possibility of cross-contamination between samples from different donors / patient families contained in separate compartments. Each compartment is supplied with filtered air and the humidity is controlled through the air-supply, rather than via a water source within the incubator. This further decreases potential sources of cross-contamination between patients. Another advantage of these separate incubator compartments is that cultures are not disturbed by the addition or removal of other cultures to and from the incubator.

Transfer hatches 2, 7, 29, 30, 32, 33, 34, 35 and 36 do not connect cabinets, but allow samples or equipment or consumables to be brought into the cabinets from outside the network. Referring now to Figure 2, transfer hatches have an outer door 28 and an inner door 26. Outer door 28 is opened to allow material to be placed inside hatch 17. Outer door 28 is then closed, and subsequently inner door 26 is opened, allowing the material to be brought into cabinet 19. This two-door system minimises exchange of air between the cabinet and the environment external to the cabinet and the cabinet remains substantially sealed.

The cabinet network is controlled by a computer based security system which can grant or restrict access to isolation cabinets and incubators.

Sample containers are assigned machine readable identification codes which are unique to a specific patient family undergoing treatment. Biological components relating to that patient are contained within sample containers labelled with the same machine readable identification code. Biological components may include sperm, oocytes, embryos or stem cell colonies. Each sample container can be tracked through the cabinets creating an electronic audit trail. In the case of embryonic stem cell derivation, patient information is removed from the identification code and a unique non-identifying donor code is used to track the samples.

Members of laboratory personnel may also be assigned an identification code which will be required to gain access to the computer system. The system allows an electronic audit trail to be created for each patient family. The system can record which doors were unlocked when and by which operator.

Figure 2 is a schematic illustration of the interior of an isolation cabinet 19 connected to a transfer hatch 17, an incubator 21 and a control unit 23. A display screen 22 inside cabinet 19 and a foot mouse 27 outside the cabinet allow the operator to interact with the computer system. Inner door 26 of transfer hatch 17 is lockable and the locking mechanism is controlled by control unit 23.

Incubator 21 comprises six independent compartments, each of which are accessible via independent, lockable inner doors. The locking mechanisms are controlled by control unit 23.

The cabinet 19 contains a scanner 20. In this example scanner 20 is an RFID scanner and machine readable identification codes on samples containers are in the form of RFID tags. Scanner 20, display screen 22, transfer hatch 17 and incubator 21 are all connected to control unit 23. Control unit 23 receives inputs from scanner 20 and operator instructions from foot mouse 27. Control unit 23 processes this input and sends appropriate outputs to incubator 21, transfer hatch 17 and display screen 22.

One or more of the cabinets may also contain an RFID writer, which allows labelling /information to be added to culture dishes. For example, in stem cell derivation, when separating out stem cell colonies from the same donor, they can be given the same base code so that the system allows them to be stored in the same incubator compartment.

As described in more detail below, control unit 23 checks that cabinet 19 contains no tagged sample containers before unlocking door 26 to allow samples or equipment to be introduced to cabinet 19. Once samples are introduced into cabinet 19, scanner 20 identifies the unique machine readable identification code associated with that sample and unlocks the door to the incubator compartment assigned to that patient family.

With reference to Figure 1, in an IVF procedure, oocytes are collected from the patient in egg collection area 1. The patient wears a wristband which is labelled with an RFID tag that is unique to that particular patient family. The RFID tag on the patient's wristband is scanned using a hand held scanner. The wristband scan prompts a scanner inside cabinet 3 to scan the contents of cabinet 3. If no RFID tagged samples are present inside cabinet 3, the inner door of transfer hatch 2 will be automatically unlocked allowing the oocyte sample to be transferred from egg collection area 1 into isolation cabinet 3 via transfer hatch 2. A member of laboratory personnel washes and counts the oocytes in cabinet 3 using the glove openings 41 to manipulate equipment inside the cabinet. The operator may add a commentary to the patient records at any point during the procedure. For example the number of oocytes collected may be added to the patient record using foot mouse 27 (fig.2), alternatively the operator may use foot mouse 27 to activate a voice recording system to add a voice commentary.

Prior to the oocyte collection procedure, dishes for receiving the washed oocytes are prepared in cabinet 6. These dishes are labelled with RFID tags that are unique to a particular patient family using an RFID writer 60. They are then placed in incubator 5 in the compartment assigned to that patient. When the patient's wristband is scanned in area 1 the door to the incubator compartment assigned to that patient in incubator 5 is unlocked. The unlocked compartment is indicated by a light on or beside the relevant incubator door. The operator removes the labelled dishes from incubator 5 via the opening in cabinet 3 and transfers the oocytes to the prepared labelled dishes. The prepared oocyte sample is then placed back inside the appropriate compartment in incubator 5. When the procedure is complete the incubator door is locked.

Cabinet 3 is now cleared of disposable items and cleaned. A new batch of oocytes may be now be introduced into cabinet 3 via transfer hatch 2. Again, the new patient's wristband is scanned in area 1 and the inside of cabinet 3 is also scanned. If any RFID tagged samples are already present in the cabinet, then the lock on the transfer hatch will not be unlocked and the new samples cannot be introduced into cabinet 3.

Sperm, prepared in an adjoining laboratory, is brought into cabinet 6 via transfer hatch 7. Sperm is held in a labelled container with an RFID tag indicating the family identity. The sperm container is placed inside transfer hatch 7. The inner door to cabinet 6 from transfer hatch 7 is locked. A scanner inside cabinet 6 scans the content of cabinet 6. Providing that there are no RFID tagged samples present inside cabinet 6, the control unit will unlock the inner door of transfer hatch 7 allowing the sperm container to be transferred into cabinet 6. Once inside cabinet 6, the sperm RFID tag is scanned by the scanner inside cabinet 6, this scan automatically unlocks the door of the compartment in incubator 5 containing the oocytes for that patient family. The sperm sample is placed inside incubator 5 in the same compartment as the oocytes and the incubator doors are then locked.

Following a period of incubation, oocytes and sperm will be mixed when the embryologist performs the insemination step. Oocytes are either mixed with sperm in cabinet 9 or sperm is injected directly in ICSI cabinet 14 or 15.

Cabinet 9 is used for carrying out a standard insemination procedure. Prior to commencing the insemination procedure, oocytes and sperm are transferred from the assigned compartment in incubator 5 to the assigned compartment incubator 8 via cabinet 6. Before this transfer the contents of cabinet 6 are scanned to ensure that cabinet 6 does not contain any RFID tagged samples. Once inside cabinet 6 the oocyte and sperm samples are re-scanned to open the compartment assigned to that family in incubator 8 and the samples are placed inside.

At cabinet 9 the embryologist uses the foot mouse 27 external to the cabinet to locate a particular patient family on a computer system which is interconnected to the control unit. A scanner inside cabinet 9 scans the content of cabinet 9. Providing that there are no RFTD tagged samples present inside cabinet 9, control unit 23 unlocks the particular incubator compartment containing that patient's oocytes and sperm samples in incubator 8. All other incubator compartments remain locked to prevent misidentification or contamination. The embryologist removes the sample containers from incubator 8 using glove openings 42. The unlocked incubator compartment is indicated by a light on or beside the relevant door. The gametes are mixed in cabinet 9 and the resulting culture is placed back inside the unlocked compartment in incubator 8. Any RFID tagged oocyte and sperm containers remaining in the cabinet may then removed for disposal via transfer hatch 33. When the procedure is complete all incubator doors are locked.

If the ICSI procedure is to be used, oocytes and sperm are transferred along the chain from incubator 5 to ICSI incubator 37 via cabinet 6. Before this transfer the contents of cabinet 6 are scanned to ensure that cabinet 6 does not contain any RFID tagged samples. Once inside cabinet 6 the samples are re-scanned to open the compartment assigned to that family in incubator 37 and the samples are placed inside.

The embryologist working at cabinet 14 locates the patient details on the computer system using the foot mouse 27. A scanner inside cabinet 14 scans the content of cabinet 14. Providing that there are no RFID tagged samples present inside cabinet 14, control unit 23 unlocks the particular incubator compartment containing that patient's oocytes and sperm samples in incubator 37. The operator removes the oocyte and sperm samples from incubator 37 and performs the ICSI procedure by injecting each oocyte with one sperm. When the insemination step is completed the resulting culture is transferred out of cabinet 14 via incubator 37 and into cabinet 6. Before this transfer the contents of cabinet 6 are scanned to ensure that cabinet 6 does not contain any RFID tagged samples. Once inside cabinet 6 the samples are re-scanned to open the compartment assigned to that family in incubator 8 and the samples are placed inside.

The day after mixing oocytes and sperm, a fertilisation check is carried out in cabinet 9. The embryologist uses the foot mouse 27 to locate a particular patient family on the computer system. A scanner inside cabinet 9 scans the content of cabinet 9. Providing that there are no RFID tagged samples present inside cabinet 9, control unit 23 unlocks the particular incubator compartment containing that patient's samples in incubator 8. All other incubator compartments in incubator 8 remain locked to prevent misidentification. Control unit 23 also unlocks the incubator compartment assigned to that patient family in incubator 10 ready for receipt of the sample at the end of the fertilisation checking step. The unlocked compartments are indicated by a light on or beside the relevant door.

Using the glove openings 42, the embryologist removes the sample container containing the oocyte/sperm culture from incubator 8 and brings it into cabinet 9. The embryologist then checks for evidence that fertilization of the oocytes has occurred. Once this step is completed, the sample container containing the culture is placed inside the unlocked compartment in incubator 10. When the procedure is complete all incubator doors are locked.

The embryo grading step of the procedure is carried out in cabinet 11. The embryologist uses the foot mouse to locate a particular patient family on the computer system. A scanner inside cabinet 11 scans the contents of cabinet 11. Providing that there are no RFID tagged samples present inside cabinet 11, control unit 23 unlocks the particular incubator compartment containing that patient's samples in incubator 10. All remaining compartments in incubator 10 remain locked to prevent misidentification. Control unit 23 also unlocks the incubator compartment assigned to that patient family in incubator 12 ready for receipt of the sample at the end of this step. The unlocked compartments are indicated by a light on or beside the relevant door.

Using the glove openings 43, the embryologist removes the sample container containing the embryo culture from incubator 10 into cabinet 11. The embryologist then grades the embryos and selects the best embryos (up to a maximum of three) for transfer into the female patient. These embryos are placed in a separate RFID tagged container for transfer into incubator 16 via cabinet 13. Prior to this transfer the contents of cabinet 13 are scanned to ensure that cabinet 13 does not contain any RFID tagged samples. Once inside cabinet 13 the embryo container is re-scanned to open the compartment assigned to that family in incubator 16 and the container is placed inside.

Surplus good quality embryos may be frozen for future treatment and the remainder may be used for research. Any embryos to be frozen are prepared for freezing in either cabinet 11 or 13. The embryos to be frozen are placed in labelled freezing containers (straws) and removed from cabinet 11 or 13 via transfer hatch 35 or 36 for freezing.

In Embryo transfer area 40, incubator 16 is connected to cabinet 39. Cabinet 39 is a Class II cabinet which has an open front and is not substantially sealed. Cabinet 39 is supplied with air which has been filtered through an HEPA filter but the air classification of this cabinet is dependent upon the background air in embryo transfer room 40. The patient is prepared in embryo transfer area 40. The patient wears a wristband with an RFID tag associated to that particular patient family. The patient's wristband is scanned using a hand held scanner. On receiving this scan control unit 23 unlocks the incubator compartment in incubator 16 containing that patient's embryos. The embryos are removed from incubator 16 and placed in cabinet 39 until the doctor is ready to transfer them to the patient.

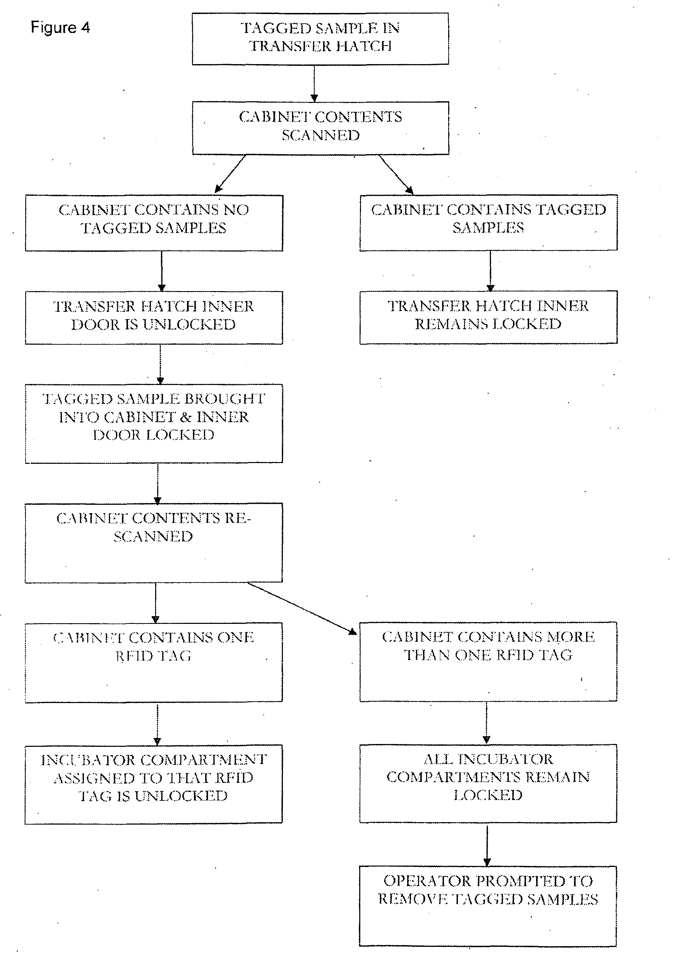

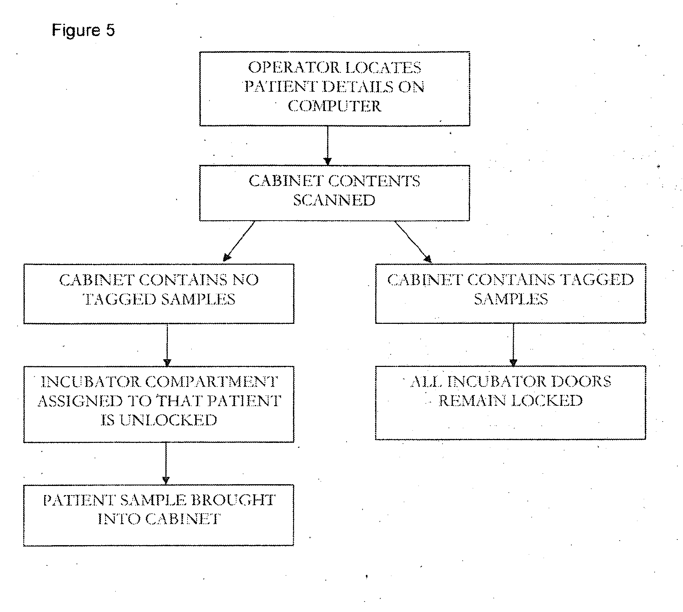

Figures 4 and 5 are flow diagrams which summarise the checking procedure carried out by the system when bringing samples into the cabinet or when removing samples from an incubator.

Referring to Figures 2 and 4, a sample, for example sperm, is introduced to transfer hatch 17 via outer door 28. The sample container is tagged with an RFID tag unique to the patient family using an RFID writer 60. The interior of cabinet 19 is scanned by RFID scanner 20 to check for tagged samples already present inside the cabinet. Scanner 20 sends an input to control unit 23.

If no tagged samples are detected inside the cabinet, control unit 23 sends an output to transfer hatch 17 unlocking door 26 allowing the sample to be brought into cabinet 19.

If a tagged sample is detected inside the cabinet, door 26 remains locked.

Once a sample has been successfully introduced to cabinet 19 from transfer hatch 17 scanner 20 re-scans the cabinet contents to check the family identity of the new tagged sample.

If the cabinet contains only one RFID tagged sample, or more than one sample with the same RFID tag, i.e. sample from the same patient family, then control unit 23 will send an output to incubator 21 unlocking the door of the incubator compartment assigned to that particular family.

If more than one sample is brought into cabinet 19 and these have different RFID tags and hence belong to more than one family, then no incubator doors are unlocked. The operator will be prompted to remove tagged samples from the cabinet until samples from only one donor /family are present. This prompt may be in the form of a visual or audible alert. The samples are then rescanned and the appropriate incubator compartment is unlocked.

Referring to Figures 2 and 5, a sample, for example patient embryos, is secured inside incubator compartment 24. In order to remove the sample from the incubator, the operator locates the patient details on a computer system linked to control unit 23 using foot mouse 27. The interior of cabinet 19 is then scanned by RFID scanner 20 to check for RFID tagged samples already present inside the cabinet. Scanner 20 sends an input to control unit 23.

If no RFID tagged samples are detected inside the cabinet, control unit 23 sends an output to incubator 21 to unlock inner door 25 and outer door 49 to allow the operator access to compartment 24. Samples from compartment 24 are then brought into cabinet 19.

If an RFID tagged sample is detected inside the cabinet, all incubator doors remain locked.

Unlocked compartments are indicated by lights on the outside of the doors. As shown in Figure 5, outer doors, for example door 54 on incubator 21 each have a light 56 on the outside of the door. Light 56 indicates which door is unlocked. Inner doors, for example door 50, also each have a light 58 on the outside of the door to indicate which compartment is unlocked. Lights 56 and 58 may be a particular colour to indicate that a particular compartment is unlocked. Alternatively lights 56 and 58 may only be switched on when that particular door is unlocked. In Figure 2, a light on outer door 49 and another light 48 on inner door 25 will indicate than compartment 24 is unlocked.

The horse shoe shaped chain of isolator cabinets enables all procedures to be carried out in a clean air environment without the need for staff to wear sterile clothes and masks. The design also minimises errors as each step of the procedure is carried out in one particular cabinet. The enclosed system also provides a constant environment which reduces cellular stress caused by fluctuations in environmental conditions during manipulations of eggs and embryos.

The electronic control system offers an active checking system which provides a physical barrier preventing samples from different patient families from coming into contact, vastly reducing the margin for error during IVF treatment.

It also provides an electronic audit trail detailing each step in the procedure. Any attempts to place dishes in the wrong compartment are recorded.

The system may also include an imaging facility to record images of the culture dishes at various stages in the procedure.

## Claims

1. Laboratory apparatus comprising at least two cabinets (3, 6, 9, 11, 13, 14, 15); at least one incubator (5, 8, 10, 12, 16, 37, 38); at least one transfer hatch (2, 7, 29, 30, 32, 33, 34, 35, 36); and means for circulating air within the apparatus, wherein the at least two cabinets are interconnected by the at least one incubator (5, 8, 10, 12, 16, 37, 38); **characterised in that** at least one incubator (5, 8, 10, 12, 16, 37, 38) comprises at least two discrete compartments (24) each accessible by independent openings (25) in either of the interconnected cabinets; and wherein the apparatus is substantially sealed against exchange of air between the cabinet and the external environment.

2. Laboratory apparatus according to claim 1, the at least one incubator (5, 8, 10, 12, 16, 37, 38) having six discrete compartments (24) each accessible by independent openings (25) in either connected cabinet.

3. Laboratory apparatus according to any preceding claim, comprising a chain of seven interconnected cabinets (3, 6, 9, 11, 13, 14, 15), the cabinets each being interconnected by an incubator (5, 8, 10, 12, 16, 37, 38) comprising at least two discrete compartments (24) each accessible by independent openings (25) in either interconnected cabinet.

4. Laboratory apparatus according to any preceding claim, wherein air is circulated through each cabinet (3, 6, 9,11, 13, 14, 15).

5. Laboratory apparatus according to claim 4, wherein air is passed through a filter before being circulated through each cabinet.

6. Laboratory apparatus according to claim 5, wherein the filter is a High Efficiency Particulate Air filter.

7. Laboratory apparatus according to any of claims 4 to 6, wherein the composition of the air is approximately 5% carbon dioxide, 5% oxygen and 90% nitrogen.

8. Laboratory apparatus according to any preceding claim, wherein the cabinets (3, 6, 9, 11, 13, 14, 15) include means to enable manipulation of equipment contained within the cabinet.

9. Laboratory apparatus according to claim 8, wherein the means to enable manipulation comprise gloves (41, 42, 43, 44, 45, 46, 47) inserted through the front wall of the cabinet (3, 6, 9, 11, 13, 14, 15).

10. Laboratory apparatus according to any preceding claim, further comprising a control unit (23) connected to each of the at least two cabinets (3, 6, 9, 11, 13, 14, 15); and at least one detector (20) for detecting machine readable information; and at least one sample container for containing a sample of a biological component; wherein the at least one container is provided with a machine readable identification code that is unique to the biological component contained within.

11. Laboratory apparatus according to claim 10, wherein the machine readable identification code may contain elements that are the same for biological components from the same donor or patient family.

12. Laboratory apparatus according to Claim 10 or 11, further comprising a display screen (22) inside the at least one cabinet (3, 6, 9, 11, 13, 14, 15).

13. Laboratory apparatus according to any of claims 10 to 12, further comprising a foot mouse (27).

14. Laboratory apparatus according to any of claims 10 to 13 further comprising voice recording means within at least one of the at least two cabinets (3, 6, 9, 11, 13, 14, 15).

15. Laboratory apparatus according to any of claims 10 to 14; wherein access openings to the at least one transfer hatch (2, 7, 29, 30, 32, 33, 34, 35, 36) and the at least one incubator (5, 8, 10, 12, 16, 37, 38) are provided with locking mechanisms.

16. Laboratory apparatus according to claim 15, wherein locking mechanisms are electronic locking mechanisms.

17. Laboratory apparatus according to claim 15 or 16, wherein locking mechanisms are controlled remotely by the control unit (23).

18. Laboratory apparatus according to any of claims 10 to 17, wherein the machine readable identification code is selected from the group comprising: barcodes or radio frequency identification tags.

19. Laboratory apparatus according to any of claims 10 to 18, wherein the at least one detector (20) is selected from the group comprising: optical scanners or radio frequency identification scanners.

20. Laboratory apparatus according to any preceding claim for use in In Vitro Fertilisation Treatment.

21. Laboratory apparatus according to any preceding claim for use in stem cell derivation.

22. A method for controlling the processing of biological components, the method comprising the steps of
(a) assigning a unique machine readable identification code to each biological component;
(b) providing a label on each sample container; and
(c) processing the samples using apparatus as claimed in any of claims 1 to 21.

23. A method according to claim 22, wherein the biological components are selected from the group comprising: sperm or oocytes, embryos or stem cells.

24. Laboratory apparatus according to any of claims 1 to 21 further comprising means for recording images of the contents of at least one of the at least two cabinets and/or the at least one incubator.

25. Laboratory apparatus according to any of claims 1 to 21 or claim 24, further comprising an alert to alert a user when samples from more than one donor or family are introduced to the same section.

## Patentansprüche

1. Laborapparatur, die Folgendes umfasst: wenigstens zwei Schränke (3, 6, 9, 11, 13, 14, 15); wenigstens einen Inkubator (5, 8, 10, 12, 16, 37, 38); wenigstens eine Transferlulce (2, 7, 29, 30, 32, 33, 34, 35, 36); und Mittel zum Zirkulieren von Luft in der Apparatur, wobei die wenigstens zwei Schränke durch den wenigstens einen Inkubator (5, 8, 10, 12, 16, 37, 38) miteinander verbunden sind; **dadurch gekennzeichnet, dass** wenigstens ein Inkubator (5, 8, 10, 12, 16, 37, 38) wenigstens zwei getrennte Fächer (24) umfasst, die jeweils durch unabhängige Öffnungen (25) in den beiden miteinander verbundenen Schränken zugängig sind; und wobei die Apparatur gegen Luftaustausch zwischen Schrank und Außenumgebung im Wesentlichen abgedichtet ist.

2. Laborapparatur nach Anspruch 1, wobei der wenigstens eine Inkubator (5, 8, 10, 12, 16, 37, 38) sechs getrennte Fächer (24) hat, die jeweils durch unabhängige Öffnungen (25) in beiden verbundenen Schränken zugängig sind.

3. Laborapparatur nach einem der vorherigen Ansprüche, die eine Kette von sieben miteinander verbundenen Schränken (3, 6, 9, 11, 13, 14, 15) umfasst, wobei die einzelnen Schränke durch einen Inkubator (5, 8, 10, 12, 16, 37, 38), der wenigstens zwei getrennte Fächer (24) aufweist, die jeweils durch unabhängige Öffnungen (25) in den verbundenen Schränken zugängig sind, miteinander verbunden sind.

4. Laborapparatur nach einem der vorherigen Ansprüche, wobei Luft durch jeden Schrank (3, 6, 9, 11, 13, 14, 15) zirkuliert wird.

5. Laborapparatur nach Anspruch 4, wobei Luft vor dem Zirkulieren durch jeden Schrank durch ein Filter geleitet wird.

6. Laborapparatur nach Anspruch 5, wobei das Filter ein HEPA-(High Efficiency Particulate Air)-Filter ist.

7. Laborapparatur nach einem der Ansprüche 4 bis 6, wobei sich die Luft aus etwa 5 % Kohlendioxid, 5 % Sauerstoff und 90 % Stickstoff zusammensetzt.

8. Laborapparatur nach einem der vorherigen Ansprüche, wobei die Schränke (3, 6, 9, 11, 13, 14, 15) Mittel zum Ermöglichen der Handhabung von in dem Schrank enthaltenen Geräten beinhalten.

9. Laborapparatur nach Anspruch 8, wobei die Mittel zum Ermöglichen der Handhabung Handschuhe (41, 42, 43, 44, 45, 46, 47) umfassen, die durch die Frontwand des Schranks (3, 6, 9, 11, 13, 14, 15) gesteckt werden.

10. Laborapparatur nach einem der vorherigen Ansprüche, die ferner Folgendes umfasst: eine Steuereinheit (23), die mit jedem der wenigstens zwei Schränke (3, 6, 9, 11, 13, 14, 15) verbunden ist; und wenigstens einen Detektor (20) zum Detektieren von maschinenlesbaren Informationen; und wenigstens einen Probenbehälter zum Aufnehmen einer Probe einer biologischen Komponente; wobei der wenigstens eine Behälter mit einem maschinenlesbaren Identifikationscode versehen ist, der für die darin enthaltene biologische Komponente eindeutig ist.

11. Laborapparatur nach Anspruch 10, wobei der maschinenlesbare Identifikationscode Elemente enthalten kann, die für biologische Proben vom selben Spender oder von derselben Patientenfamilie dieselben sind.

12. Laborapparatur nach Anspruch 10 oder 11, die ferner einen Anzeigeschirm (22) in dem wenigstens einen Schrank (3, 6, 9, 11, 13, 14, 15) umfasst.

13. Laborapparatur nach einem der Ansprüche 10 bis 12, die ferner eine Fußmaus (27) umfasst.

14. Laborapparatur nach einem der Ansprüche 10 bis 13, die ferner ein Sprachaufzeichnungsmittel in wenigstens einem der wenigstens zwei Schränke (3, 6, 9, 11, 13,14, 15) umfasst.

15. Laborapparatur nach einem der Ansprüche 10 bis 14, wobei Zugangsöffnungen zu der wenigstens einen Transferluke (2, 7, 29, 30, 32, 33, 34, 35, 36) und dem wenigstens einen Inkubator (5, 8, 10, 12, 16, 37, 38) mit Verriegelungsmechanismen versehen sind.

16. Laborapparatur nach Anspruch 15, wobei Verriegelungsmechanismen elektronische Verriegelungsmechanismen sind.

17. Laborapparatur nach Anspruch 15 oder 16, wobei Verriegelungsmechanismen von der Steuereinheit (23) ferngesteuert werden.

18. Laborapparatur nach einem der Ansprüche 10 bis 17, wobei der maschinenlesbare Identifikationscode aus der Gruppe ausgewählt wird, die Barcodes oder Radiofrequenz-Identifikationsmarken beinhaltet.

19. Laborapparatur nach einem der Ansprüche 10 bis 18, wobei der wenigstens eine Detektor (20) aus der Gruppe ausgewählt wird, die optische Scanner oder Radiofrequenz-Identifikationsscanner beinhaltet.

20. Laborapparatur nach einem der vorherigen Ansprüche für die Verwendung bei In-vitro-Fertilisationsbehandlungen.

21. Laborapparatur nach einem der vorherigen Ansprüche für die Verwendung bei der Stammzellengewinnung.

22. Verfahren zum Steuern der Verarbeitung von biologischen Komponenten, wobei das Verfahren die folgenden Schritte beinhaltet:
(a) Zuordnen eines eindeutigen maschinenlesbaren Identifikationscodes zu jeder biologischen Komponente;
(b) Versehen jedes Probenbehälters mit einem Etikett; und
(c) Verarbeiten der Proben mit einer Apparatur nach einem der Ansprüche 1 bis 21.

23. Verfahren nach Anspruch 22, wobei die biologischen Komponenten aus der Gruppe ausgewählt sind, die Sperma oder Oozyten, Embryos oder Stammzellen beinhaltet.

24. Laborapparatur nach einem der Ansprüche 1 bis 21, die ferner Mittel zum Aufzeichnen von Bildern des Inhalts von wenigstens einem der wenigstens zwei Schränke und/oder dem wenigstens einen Inkubator umfasst.

25. Laborapparatur nach einem der Ansprüche 1 bis 21 oder Anspruch 24, die ferner einen Alarm beinhaltet, um einen Benutzer zu alarmieren, wenn Proben von mehr als einem/r Spender oder Familie in dieselbe Sektion eingeleitet werden.

## Revendications

1. Un appareil de laboratoire comprenant au moins deux coffrets (3, 6, 9, 11, 13, 14, 15), au moins un incubateur (5, 8, 10, 12, 16, 37, 38), au moins un sas de transfert (2, 7, 29, 30, 32, 33, 34, 35, 36), et un moyen de circulation d'air à l'intérieur de l'appareil, où les au moins deux coffrets sont interconnectés par le au moins un incubateur (5, 8, 10, 12, 16, 37, 38), **caractérisé en ce que** le au moins un incubateur (5, 8, 10, 12, 16, 37, 38) comprend au moins deux compartiments discrets (24), chacun d'eux étant accessible par des ouvertures indépendantes (25) dans l'un ou l'autre des coffrets interconnectés, et où l'appareil est sensiblement étanche à un échange d'air entre le coffret et l'environnement externe.

2. Un appareil de laboratoire selon la Revendication 1, le au moins un incubateur (5, 8, 10, 12, 16, 37, 38) possède six compartiments discrets (24), chacun d'eux étant accessible par des ouvertures indépendantes (25) dans l'un ou l'autre des coffrets connectés.

3. Un appareil de laboratoire selon l'une quelconque des Revendications précédentes, comprenant une chaîne de sept coffrets interconnectés (3, 6, 9, 11, 13, 14, 15), les coffrets étant tous interconnectés par un incubateur (5, 8, 10, 12, 16, 37, 38) comprenant au moins deux compartiments discrets (24), chacun d'eux étant accessible par des ouvertures indépendantes (25) dans l'un ou l'autre des coffrets interconnectés.

4. Un appareil de laboratoire selon l'une quelconque des Revendications précédentes, où de l'air est circulé au travers de chaque coffret (3, 6, 9,11, 13, 14, 15).

5. Un appareil de laboratoire selon la Revendication 4, où de l'air est passé au travers d'un filtre avant d'être circulé au travers de chaque coffret.

6. Un appareil de laboratoire selon la Revendication 5, où le filtre est un filtre à air particulaire haute efficacité.

7. Un appareil de laboratoire selon l'une quelconque des Revendications 4 à 6, où la composition de l'air est approximativement de 5 % de dioxyde de carbone, 5 % d'oxygène et 90 % d'azote.

8. Un appareil de laboratoire selon l'une quelconque des Revendications précédentes, où les coffrets (3, 6, 9, 11, 13, 14, 15) comprennent un moyen de permettre une manipulation de l'équipement contenu à l'intérieur du coffret.

9. Un appareil de laboratoire selon la Revendication 8, où le moyen de permettre une manipulation comprend des gants (41, 42, 43, 44, 45, 46, 47) insérés au travers de la paroi avant du coffret (3, 6, 9, 11, 13, 14, 15).

10. Un appareil de laboratoire selon l'une quelconque des Revendications précédentes, comprenant en outre une unité de commande (23) raccordée à chacun des au moins deux coffrets (3, 6, 9, 11, 13, 14, 15), et au moins un détecteur (20) destiné à détecter des informations lisibles par ordinateur, et au moins un récipient à échantillon destiné à contenir un échantillon d'un composant biologique, où le au moins un récipient est muni d'un code d'identification lisible par ordinateur qui est unique au composant biologique contenu dans celui-ci.

11. Un appareil de laboratoire selon la Revendication 10, où le code d'identification lisible par ordinateur peut contenir des éléments qui sont les mêmes pour des composants biologiques provenant de la même famille de patients ou donneurs.

12. Un appareil de laboratoire selon la Revendication 10 ou 11, comprenant en outre un écran d'affichage (22) dans le au moins un coffret (3, 6, 9, 11, 13, 14, 15).

13. Un appareil de laboratoire selon l'une quelconque des Revendications 10 à 12, comprenant en outre une souris à pédale (27).

14. Un appareil de laboratoire selon l'une quelconque des Revendications 10 à 13 3 comprenant en outre un moyen d'enregistrement vocal à l'intérieur d'au moins un des au moins deux coffrets (3, 6, 9, 11, 13, 14, 15).

15. Un appareil de laboratoire selon l'une quelconque des Revendications 10 à 14, où les ouvertures d'accès au au moins un sas de transfert (2, 7, 29, 30, 32, 33, 34, 35, 36) et au au moins un incubateur (5, 8, 10, 12, 16, 37, 38) sont équipées de mécanismes de verrouillage.

16. Un appareil de laboratoire selon la Revendication 15, où les mécanismes de verrouillage sont des mécanismes de verrouillage électroniques.

17. Un appareil de laboratoire selon la Revendication 15 ou 16, où les mécanismes de verrouillage sont commandés à distance par l'unité de commande (23).

18. Un appareil de laboratoire selon l'une quelconque des Revendications 10 à 17, où le code d'identification lisible par ordinateur est sélectionné dans le groupe comprenant :
codes à barres ou étiquettes d'identification par radiofréquence.

19. Un appareil de laboratoire selon l'une quelconque des Revendications 10 à 18, où le au moins un détecteur (20) est sélectionné dans le groupe comprenant : scanners optiques ou scanners d'identification par radiofréquence.

20. Un appareil de laboratoire selon l'une quelconque des Revendications précédentes destiné à une utilisation dans un traitement de fertilisation in vitro.

21. Un appareil de laboratoire selon l'une quelconque des Revendications précédentes destiné à une utilisation dans la dérivation de cellules souches.

22. Un procédé de commande du traitement de composants biologiques, le procédé comprenant les opérations suivantes :
(a) l'attribution d'un code d'identification unique lisible par ordinateur à chaque composant biologique,
(b) la fourniture d'une étiquette sur chaque récipient à échantillon, et
(c) le traitement des échantillons au moyen d'un appareil selon l'une quelconque des Revendications 1 à 21.

23. Un procédé selon la Revendication 22, où les composants biologiques sont sélectionnés dans le groupe comprenant : sperme ou ovocytes, embryons ou cellules souches.

24. Un appareil de laboratoire selon l'une quelconque des Revendications 1 à 21 1 comprenant en outre un moyen d'enregistrement d'images des contenus d'au moins un des au moins deux coffrets et/ou du au moins un incubateur.

25. Un appareil de laboratoire selon l'une quelconque des Revendications 1 à 21 ou 24, comprenant en outre une alerte destinée à alerter un utilisateur lorsque des échantillons provenant de plus d'un donneur ou famille sont introduits dans la même section.
